# EUROPEAN PATENT SPECIFICATION

(11) **EP 1 850 780 B1**
(45) Date of publication and mention of the grant of the patent: **20.06.2012**
(21) Application number: 06704837.1
(22) Date of filing: 10.02.2006
(51) Int. Cl.: A61B 18/14

(54) **AN ELECTROSURGICAL PROBE**
ELEKTROCHIRURGISCHE SONDE
SONDE ELECTROCHIRURGICALE

(30) Priority: 11.02.2005 AU 2005900641; 16.02.2005 AU 2005900726
(43) Date of publication of application: 07.11.2007
(73) Proprietor: MacKay, Dale Victor, Carlton North, VIC 3054 (AU)
(72) Inventor: MacKay, Dale Victor, Carlton North, VIC 3054 (AU)
(74) Representative: Townsend, Victoria Jayne
(86) International application number: PCT/AU2006/000159
(87) International publication number: WO 2006/084316

(56) References cited:
- EP-A- 1 293 169
- EP-A1- 1 561 430
- WO-A-98/25530
- WO-A-03/028540
- WO-A1-01/95819
- WO-A2-03/028540
- RU-C2- 2 190 357
- US-A- 5 697 281
- US-A1- 2002 022 870
- US-A1- 2002 183 744
- US-A1- 2003 109 869
- US-A1- 2004 044 342
- US-A1- 2004 044 342
- US-A1- 2004 138 658
- US-A1- 2004 167 512
- US-B1- 6 197 026

## Description

### Field of the Invention

The present invention relates to electrosurgery and to probes for use with electrosurgical devices. It relates particularly but not exclusively to probes and probe attachments for use in endoscopic electrosurgery.

### Background to the Invention

Electrosurgery uses high frequency energy to cauterise, burn or ablate tissue whilst minimising bleeding. Electrosurgery can be practiced using bipolar or monopolar modality. In a bipolar mode, electrosurgical equipment passes a current between two electrodes resulting in an arc between the electrodes. Tissue located between the electrodes is vaporised or cauterised by heat generated by the electrical discharge thereby creating an electrosurgical cut or cauterisation of the tissue.

In monopolar modality, the patient forms part of the electric circuit. Thus high frequency current applied by a single discharge electrode travels through the patient's body to a return electrode attached elsewhere on the patent which is in turn connected to ground. In an isolated circuit, the patient return electrode is connected to the electrosurgical generator unit thus completing the electric circuit with a floating electrical potential between the discharge and the return electrodes. This reduces the risk of accidental patient bums at non-surgical sites that can be caused by the applied current seeking an alternate path to ground.

A range of tissue effects can be created using electrosurgical devices. These include cutting, fulguration, desiccation, coagulation and ablation. In cutting, a lower voltage, high duty cycle RF (radio frequency) current is focussed at the site creating intense heat. This causes vaporisation of tissue accompanied by hemostasis in which bleeding at the site is minimised or prevented. Fulguration is usually performed with a modulated power source at a reduced duty cycle such that the high frequency energy is applied to the site in short bursts. This dehydrates, coagulates and chars the tissue over a larger area, also sealing the tissue. For desiccation, the electrode is usually in direct contact with the target tissue thereby reducing the current concentration. Thus, less heat is generated causing the cells to dehydrate and form a coagulum, rather than vaporising as is the case in cutting. In each mode, different effects can be produced by varying the waveform used to energise the discharge electrode and by altering the distance between the discharge electrode and the target tissue.

Control of the electrosurgical energy applied to the target tissue has been improved by introducing a gas stream into the discharge path of the electrode. In gas enhanced electrosurgery, an easily ionised inert gas such as argon or helium is introduced to the surgical site and used to form a shroud around the discharge electrode. This has the effect of moderating the electrical discharge. The gas also blankets the surgical site thereby suppressing charring of tissue in the area. Use of ionisable gas in this way is also thought to reduce heating of the discharge electrode thus inhibiting electrode destruction and eschar build up.

The inert gas provides a more conductive medium than oxygen and other gases which would ordinarily exist around the discharge electrode. Thus, current discharged from the electrode seeks the path of least resistance, ionising the argon stream and arcing to the tissue bed. This results in better directional delivery of the RF current to the target tissue and causes less damage than conventional RF current. Moreover, since the ionisable gas displaces oxygen in the discharge path there is less carbonisation and therefore less eschar build up, accompanied by decreased smoke plume.

Advantageously, the level of hemostasis created using electrosurgery reduces the amount of trauma caused to the tissue during the procedure. This facilitates faster wound healing with reduced propensity for complications arising from infection. In the early 1990's the concept of utilising electrosurgical techniques through a flexible endoscope was investigated. Flexible endoscopes are used to view internal organs and tissues of a patient. Procedures using flexible endoscopes are usually performed by gastroenterologists, surgeons and respiratory physicians to explore and treat organs including the gastrointestinal tract and bowels, bronchioles and lungs, and joints and other tissues which can be viewed laparoscopically.

Endoscopic electrosurgery has been used to achieve dissection and hemostasis. To treat lesions on the mucosal wall of the gut, for example, the surgeon holds the discharge electrode tip at a distance from the target tissue and activates the electrode to generate an arc to the tissue. Hence the desired tissue coagulation or fulguration is achieved. There is, however, an associated risk of burning too deeply into the mucosal wall because it is harder to control the electrosurgical discharge endoscopically than it is in open surgery procedures. Delivering the RF current through a stream of inert gas such as argon improves control over delivery of the RF energy. Therefore gas enhanced electrosurgery is common in endoscopic procedures.

However, such procedures are not performed without risk, and the known electrosurgical probes are not well suited for use in the gastrointestinal tract, for example, because during most endoscopic procedures the patient is heavily sedated and can move around due to breathing or discomfort causing the target tissue to move during treatment. This increases the likelihood of the probe tip coming into contact with the mucosal wall and during delivery of gas and activation of the electrode this can cause perforation of the mucosal wall and gas embolism in the target tissue. Accordingly, use of these probes in endoscopic procedures is potentially dangerous. When these probes are used endoscopically the surgeon's technique is vital to ensure that the probe does not make contact with the mucosal wall while the electrode is activated.

Experienced surgeons may develop a feel for the patient's movements and move the probe away from the tissue surface and/or de-activate the electrode (e.g. by releasing a foot-operated pedal) in anticipation of the probe tip contacting the mucosal wall. However, this is not a fail-safe approach and even the most experienced surgeons make occasional contact between the activated electrode and the tissue surface putting the patient at risk of gas embolism. It would be desirable to provide a probe which is safe for use in endoscopic electrosurgery.

It is an object of the present invention to overcome or ameliorate one or more of the disadvantages of the prior art, or at least to provide a useful alternative.

The discussion of the background to the invention included herein including reference to documents, acts, materials, devices, articles and the like is intended to explain the context of the present invention. This is not to be taken as an admission or a suggestion that any of the material referred to was published, known or part of the common general knowledge in Australia as at the priority date of any of the claims.

### Summary of the Invention

In a first aspect of the present invention there is provided a probe for use in electrosurgery, the probe including:
(a) a lumen having a proximal end and a distal opening;
(b) an electrode within the lumen, the electrode having a proximal end connectable to a high frequency current source and a discharge end recessed within the lumen; and
(d) a gas outlet in a distal region of the lumen for releasing gas pressure;
wherein during use, when the distal opening of the lumen contacts target tissue while the electrode is activated gas pressure within the lumen is released through the gas outlet thus avoiding gas embolism.

The distal opening may be shaped to create a desired treatment effect in the target tissue. Preferably, the distal opening is oblique to facilitate treatment of a larger area of target tissue. This also enables a surgeon to have better directional control of the high frequency current when applied to target tissue endoscopically. When the distal opening is oblique it is preferred that the gas outlet is positioned such that the likelihood of both the distal opening and the gas outlet contacting the target tissue simultaneously is minimised. Thus, the gas outlet is preferably located in a wall of the lumen substantially opposing the distal opening.

Whilst the gas outlet may be provided anywhere in the distal region of the lumen which permits release of gas pressure within the lumen, it is preferably provided adjacent the discharge end of the electrode and may be located just distal of the discharge end of the electrode. Whilst it may be sufficient to provide one gas outlet, it is possible that more than one gas outlet may be provided to release pressure created by gas within the lumen. Where more than one gas outlet is provided, the plurality of outlets may be smaller to provide a similar sum total gas release as a single, larger gas outlet. The outlet may take any suitable shape including round or oval, for example. Alternatively, the gas outlet may be provided by a series of holes or slots.

Preferably, a distal region of the lumen includes a transparent portion. The transparent portion may be provided in the form of a transparent window, or the distal region of the probe may be formed entirely of a transparent material. Preferably the transparent portion is ceramic, although other suitable insulating materials with heat resistant properties may be used.

In one embodiment, the distal opening has a low coefficient of friction coating for suppressing eschar build up and also to prevent tissue, blood and mucous from adhering during use. The distal region may also include a marking to indicate the orientation of the distal opening when in use. In one embodiment, the probe further includes pivot means for enabling the distal opening to maintain contact with the target tissue during movement of the probe or target tissue. Preferably, the gas within the lumen is argon gas although helium or other ionisable gases may be used.

In a preferred embodiment, a distal portion of the electrode is tungsten with a highly conductive coating at its working tip. One suitable coating is silver. The probe is suitable for use within a working channel of a flexible endoscope. Such endoscopes include devices adapted for use in the gastrointestinal tract and/or respiratory system, and devices used in laparoscopic surgery.

Target tissue may be treated using the probe by contacting the distal opening to the target tissue during activation of the electrode. In use, the distal opening may also be drawn along the target tissue surface during activation of the electrode to achieve ablation of a larger region of tissue. This may incorporate a sweeping motion.

According to another aspect of the present invention, there is provided an attachment for an electrosurgical probe having an electrode, the attachment including:
(a) a lumen having a proximal end connectable to the probe and a distal opening being a working tip; and
(b) a gas outlet in the lumen for releasing gas pressure;
wherein upon attachment to the probe, the electrode is recessed within the lumen, and wherein during activation of the electrode, when the working tip contacts target tissue gas pressure within the lumen is released through the gas outlet thus avoiding gas embolism.

The gas outlet may be provided just distal of the discharge electrode. The distal opening may be shaped to create a desired treatment effect in the target tissue. Preferably, the distal opening is oblique. Preferably, the gas outlet is positioned so as to minimise the risk of the gas outlet and distal opening contacting the tissue simultaneously. Thus, the gas outlet is preferably provided in a wall of the lumen substantially opposing the oblique distal opening.

In one embodiment, the probe attachment includes transparent window portion although the attachment itself may be transparent. The probe attachment may be manufactured from a ceramic material, or any other insulator which can withstand high temperatures without deforming or being otherwise affected. In one embodiment the distal opening has a low coefficient of friction coating to suppress eschar build up during use and to prevent blood, mucous and tissue from adhering to the working tip.

In one embodiment, the attachment includes a marking on the lumen to indicate the orientation of the distal opening when in use. The attachment may also include pivot means for enabling the distal opening to maintain contact with the target tissue during movement of the probe or target tissue. The pivot means may take any suitable form such as a swivel or other rotatable connection.

Preferably, the probe attachment is suitable for use with a probe adapted for use within a working channel of a flexible endoscope. Such probe may be used to treat the gastrointestinal tract or respiratory system, for example.

According to another of its aspects, the present invention provides an endoscopic electrosurgery probe including a flexible lumen having a proximal end connectable to a source of ionisable gas and a distal opening, an electrode having a proximal end attachable to a high frequency current source and a discharge tip recessed within a distal region of the lumen, the lumen also having a gas outlet near the electrode discharge tip, wherein the probe is activated by shrouding the electrode in ionisable gas and applying high frequency current to the electrode, and wherein in use, gas pressure within the lumen is released through the gas outlet when the distal opening contacts target tissue during activation of the electrode.

### Brief description of the drawings

The present invention will now be described in greater detail with reference to the accompanying drawings. It is to be understood that the particularity of the accompanying drawings does not supersede the generality of the preceding description of the invention.
Fig. 1 illustrates part of a probe according to an embodiment of the invention.
Fig. 2 illustrates the tip of the probe of Fig. 1 in use, and having a transparent distal region.
Fig. 3 illustrates a probe attachment according to another embodiment of the invention.
Fig. 4 is a top view of a probe according to another embodiment of the present invention, having pivot means and a marking to indicate the orientation of the distal opening.
Fig. 5 is an exploded view of the probe of Fig. 4 showing the pivot means.

### Detailed Description

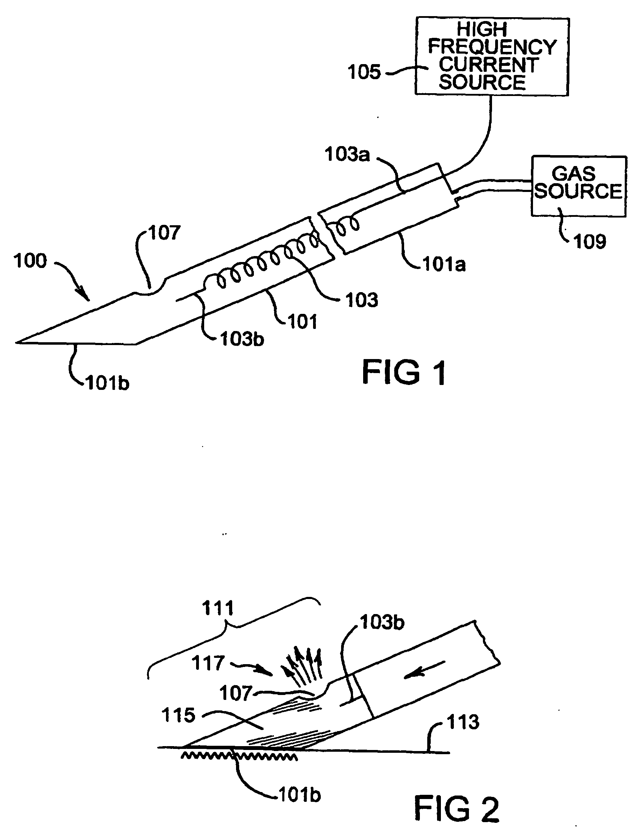

Referring firstly to Fig 1, a probe indicated generally at 100 is shown for use in electrosurgery according to an embodiment of the invention. A lumen 101 has a proximal end 101 a and a distal opening 101 b. The break in the lumen between the proximal and distal ends indicates that the lumen is of variable length. An electrode 103 is provided within the lumen. The electrode has a proximal end 103a connectable to a high frequency current source 105, and a discharge end 103b recessed within the lumen distally. A gas outlet 107 is provided in a distal region of the lumen for releasing gas pressure during use of the probe. Thus during use, when the distal opening 101 b of the lumen contacts target tissue while the electrode 103 is activated with high frequency current from source 105, gas pressure within the lumen is released through the gas outlet 107 thereby avoiding the tendency to develop a gas embolism in the target tissue.

The probe 100 is preferably a long flexible tube connectable to a base unit which includes a high frequency current source 105. The current source may operate at radio frequency (RF) for example, although other high frequencies which may not be considered to be within the RF range may also be suitable. Preferably, the base unit also has a source of ionisable gas 109. Suitable base units include electrosurgical generator units and argon delivery systems of the kind manufactured by ConMed, Valleylab, Erbe and Soring. One particularly suitable type of base unit for use with the inventive probe is the ConMed ABC although other existing units may be equally suitable.

Connection of the proximal end of the lumen with a gas source 109 enables a stream of gas to flow through the lumen and exit through the distal opening. Upon activation of the electrode the RF current flows through and ionises the gas, utilizing it as a conduit to deliver the energy to the target tissue. Preferably, the gas is an inert ionisable gas such as argon, although other gases including helium may be used.

Electrode 103 extends inside lumen 101 and terminates before distal opening 101 b. Preferably, the electrode is a flexible wire having a tungsten tip at its discharge end, although the entire electrode may be made from tungsten. Tungsten provides an effective material from which an arc of RF energy can be discharged due to its high melting temperature and heat resistant properties. The working tip of the electrode may be coated with a highly conductive material. One suitable conductive material is silver. Silver is about 40% more conductive than other elements which are used to manufacture electrodes. Coating the discharge tip of the electrode with silver creates a highly conductive interface between the tungsten electrode and the ionisable gas, thereby assisting in initiation of current arcing and ionisation of the gas.

In prior art electrosurgical probes, RF current from the electrode ionises the gas shrouding the electrode creating an arc to the target tissue. It is the arc of RF energy which produces the desired ablation and haemostasis effects. Using these probes, ablation of target tissues is not sharply focussed because the stream of ionisable gas spreads and disperses as it exits the probe tip, forming a blanket over the target tissue. These probes are intended for use in a noncontact mode. That is, with a gap between the electrode and the underlying tissue so that an arc can form across the gap and ablate the tissue.

This does not create difficulty in most electrosurgical procedures where the patient is anaesthetised and the target tissue is motionless and accessible. However, maintaining the requisite arc-inducing gap between the probe and the target tissue is more challenging in endoscopic procedures. In endoscopic electrosurgery performed in the gastrointestinal (GI) tract, for example, complications arise because the patient is usually mildly sedated and often moves during the procedure due to breathing, discomfort and internal bodily functions. This creates difficulties for the surgeon because the tissue to be ablated becomes a moving target.

Thus it can be difficult to maintain a gap between the probe and the target tissue while the electrode is activated, and the tip of the probe can inadvertently become buried into the tissue wall. Contact between the tissue wall and the probe can cause serious complications during activation of the electrode as the tissue wall can become perforated by the gas stream exiting the probe. Contact between the tissue wall and the probe can also create a potentially life threatening situation as the risk of embolism developing is significant. It is considered a contraindication to activate these prior art probes in contact-mode. It follows that it is a contraindication to use the prior art probes endoscopically in gastroscopy, colonoscopy, bronchoscopy and other procedures where the likelihood of contact between the probe and the target tissue is high.

The present invention addresses this problem by providing a probe 100 which is suitable for use in contact with the target tissue. This is due to the provision of gas outlet 107 which provides a vent for gas pressure to escape from the lumen 101 when the distal opening 101b is in contact with the target tissue during activation of the electrode 103 as shown in Fig. 2. Instead of the activated probe pumping gas into the mucosal wall, gas pressure within the lumen is released through the gas outlet 107 removing the risk of perforation by gas pressure and gas embolism. The gas outlet must be sufficiently large to allow gas to exit easily when the distal opening is in contact with the contact tissue. For most lumens, approximately 1 mm in diameter has been found adequate.

The gas outlet provides a safety feature which permits activation of the electrode while the distal opening 101 b of the lumen is in contact with or buried in the target tissue. Accordingly, during use the distal opening of the lumen may be rested on the target tissue allowing accurate, controlled ablation. Since the distal opening is in contact with the tissue, the RF energy is confined by the lumen walls and arcing to adjacent tissues and structures is minimised. This results in less indiscriminate exposure of the surrounding tissue to the applied RF current than the prior art probes.

Preferably, the gas outlet 107 is located slightly distal of the electrode 103, with the broadest region of the opening between the discharge end 103b of the electrode and the distal opening 101 b of the lumen. Advantageously, the outlet can also be used as a washing port for cleaning the electrode and the distal region of the lumen to dislodge and remove blood, mucous and debris which may build up during use of the probe. The lumen can be cleaned by syringing the distal region through the gas outlet 107 with saline, water or a cleaning fluid. Alternatively, the distal region of the lumen can be cleaned by use of a stiletto inserted through the gas outlet to dislodge any debris or tissue inside the lumen.

Preferably, the lumen has an external diameter of less than about 2.4 mm and can be inserted into the working channel of a flexible endoscope of the kind routinely used in gastroscopy, colonoscopy and bronchoscopy procedures. In use, the distal end of the lumen protrudes beyond the distal end of the working channel, outside the endoscope.

Fig. 2 illustrates the working end of a probe in use, according to an embodiment of the invention. Inside the distal region 111 of the lumen, argon gas 115 shrouds the electrode 103 providing a path of least resistance for the RF current to flow from the discharge end of the electrode 1 03b to the target tissue 113. Electrons complete the electrical circuit by returning to the patient return electrode located elsewhere on the patient. Because the probe is suitable for use with the distal opening 101 b in contact with the target tissue, the applied current is confined by the lumen walls and does not spread over the target tissue to the surrounding areas.

The ionisable gas can be accurately and directionally controlled to treat a specific target site during activation of the electrode, with the outlet 107 providing an escape route for gas pressure 117 during treatment. Release of gas pressure through the gas outlet prevents a build up of pressure on the mucosal wall which occurs during use of the prior art probes. This alleviates the potential for the inert gas flow to perforate the tissue wall, enabling the surgeon to perform ablation of tissues without the concerns of tissue contact. The target tissue is ablated with precision and safety without risk of accidentally causing gas embolism.

The distal opening may be any suitable shape. However, in a preferred embodiment, the distal opening 101b is an oblique opening of the kind illustrated in the attached drawings. This has been found to be ideal particularly for endoscopic procedures because the surgeon is able to direct the RF energy at the tissue wall with the lumen resting along the tract (e.g. the mucosal wall). The orientation of the distal opening can be controlled for precise delivery of RF current and ablation. Also, the oblique opening permits a greater surface area of contact when directing RF energy from the electrode. This can, where desired, produce shallower ablation which is useful for surgeons working on areas of the tract that are thin walled and particularly prone to perforation, such as the Cecum.

Because the distance between the discharge electrode and the tissue remains substantially constant when the distal opening is in contact with the tissue during treatment, more consistent ablation depths can be achieved. The distal opening may be drawn along the surface of the target tissue during activation of the electrode, enabling a larger area of tissue to be treated with a substantially consistent delivery of RF energy. A sweeping motion may be used along the tissue wall and this may further assist with clearing debris from the distal region of the lumen during use. Whilst the present invention is suitable for use while maintaining contact between the distal opening and the target tissue, it may also be used with a gap between the distal opening and the target tissue.

In a particularly preferred embodiment, a portion of the distal region of the lumen is transparent. The transparent portion may be in the form of a transparent window. In a particularly preferred embodiment, the entire distal region 111 of the lumen is transparent, as illustrated in Fig. 2. This enables the surgeon to view the discharge end 103b of the electrode and monitor its condition. The surgeon can also view the inside of the distal region of the lumen ensure that effective ionisation is taking place and that the lumen is clear of blood, mucous and debris. The transparent portion also allows the surgeon to view the target tissue and the electrode during operation, and direct the argon beam at the area of tissue to be treated very accurately, and substantially without adversely affecting the nearby tissue.

The transparent portion also enables the surgeon to ascertain quickly whether the probe tip including the distal opening (and gas outlet) is clear of blood, mucous and debris. This is advantageous because debris on the electrode inhibits effective ionisation of the gas. Utilising the transparent tip, the surgeon can monitor the condition of the electrode while it is in use thereby avoiding activation without ionisation, resulting in preservation of the discharge end of the electrode. This also reduces the severe pain and discomfort caused by the prior art probes which can cause distension of the patient's gastrointestinal tract (e.g. abdomen or colon) when gas is not effectively ionised. Further, it eliminates the time wastage associated with treatment using the prior art probes which must be stopped periodically to inspect the probe and ensure that there is no debris or eschar build up on the electrode.

Preferably, the transparent portion is made from a ceramic insulating material, although other insulating materials which have high melting temperatures may be used. In a particularly preferred embodiment, the distal opening 101b is coated with a low coefficient of friction substance such as Teflon® to suppress eschar build up on the tip. A Teflon® (or other low coefficient of friction) coating also reduces the propensity for tissue to adhere to the distal opening during operation, thereby reducing inadvertent tissue damage resulting from use of a probe tip roughened by adhered tissue or the like. This is particularly valuable where a sweeping motion is being used to treat a larger region of target tissue.

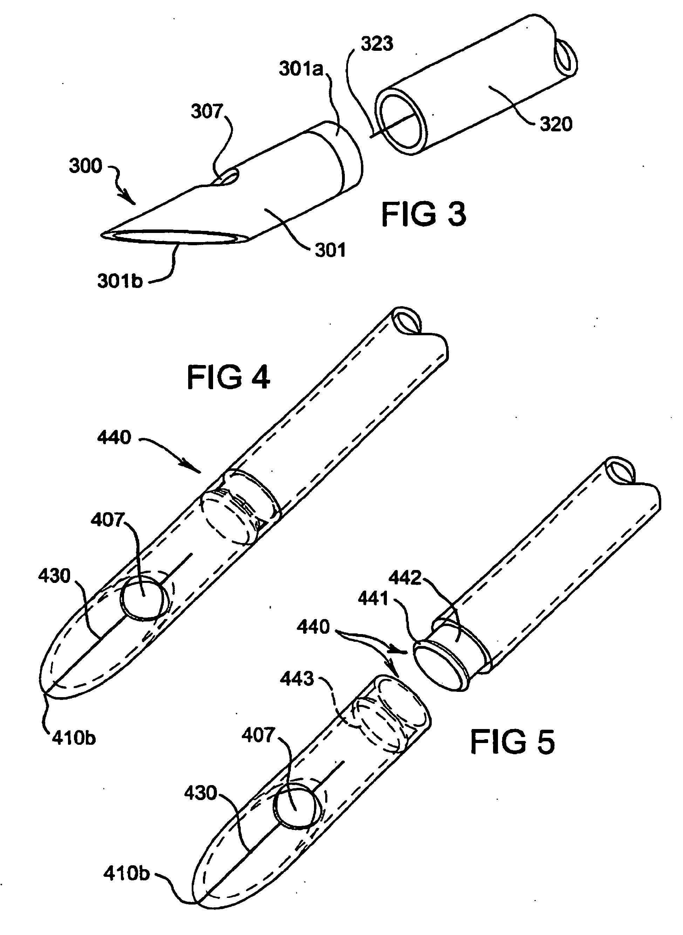

In one arrangement, the distal region of the probe includes a marker to indicate the orientation of the distal opening. In the example illustrated in Fig. 4 and Fig. 5, the marker is a line 430 which extends from the furthest point of the distal opening 410b, through the gas outlet 407 and terminates just proximal of the gas outlet. Generally, this side of the probe is in view by the surgeon operating the endoscope while using the probe with the distal opening in contact with the target tissue.

In one embodiment, the probe includes pivot means for rotating the distal opening relative to the lumen, to maintain contact between the target tissue and the distal opening during movement of the probe. The pivot means may take any suitable form and in one embodiment is a swivel or other rotatable coupling with which the distal opening rotates independently of the probe body. This enables the surgeon to align or position the distal opening by resting the it on the target tissue and allowing the end of the probe to swivel into the correct position to interface with the tissue without having to manipulate the probe.

One example of a pivot means is illustrated in Fig. 4 and Fig. 5 although it is to be understood that other pivot means can be incorporated as would be known to the person skilled in the art. In Fig. 4 and Fig. 5, swivel coupling 440 has, on one side, a neck 442 which terminates in a flange 441. On the other side, a groove 443 (shown in broken lines) is provided on the inside of the coupling to receive the flange 441 which freely rotates in clockwise and counter-clockwise directions.

Now referring to Fig. 3, there is shown a probe attachment 300 for an electrosurgical probe 320 having an electrode 323. The probe attachment has a lumen 301 having a proximal end 301 a connectable to the electrosurgical probe and a distal opening 301 b being a working tip. Preferably the electrosurgical probe is a gas enhanced probe which, during operation, shrouds electrode 323 with an inert ionisable gas such as argon or helium.

The probe attachment has a gas outlet 307 which releases gas pressure during use, in the same way that gas outlet 107 in probe 100 releases gas pressure. The probe attachment is configured such that, when attached to probe 320, the discharge end of electrode 323 is recessed within the lumen. In this manner, when the electrode is activated an arc forms inside the probe attachment between the electrode and the target tissue. When the working tip contacts the tissue, upon activation of the electrode gas pressure within the lumen is released through the gas outlet. This avoids perforating the tissue and also significantly reduces the risk of gas embolism.

In a manner similar to probe 100, the distal opening 301 b of probe attachment 300 may take any shape, but is preferably shaped to create a desired treatment effect in the target tissue. In a preferred embodiment, the distal opening is an oblique opening, formed by terminating the lumen on an angle as illustrated in Figure 3. This produces an increased treatment area and aids in directing the RF energy from the distal opening to the tissue mucosa when used endoscopically such as in the upper or lower gastrointestinal tracts. The treatment area can be varied by varying the angle at which the distal opening is formed. The greater the oblique opening, the larger the working. This can be advantageous when endeavouring to ablate a larger surface area of tissue such as Barrett's oesophagus.

When the opening is oblique it is preferred that the gas outlet is located such that there is very little chance of both the distal opening and the gas outlet contacting or becoming buried in the target tissue simultaneously, even when the device is used endoscopically. Accordingly, the gas opening is preferably provided in a wall of the lumen substantially opposing the oblique distal tip. By arranging the distal opening 301 b and gas outlet 307 in this way, the risk of tissue perforation or gas embolism is minimised. Preferably the outlet is provided just distal of the discharge electrode.

The probe attachment 300 may include a transparent portion. This may be in the form of a window through which a surgeon can view the interior of the probe attachment and electrode tip using an endoscope camera. In a preferred embodiment, substantially all of the probe attachment is transparent. This provides the same advantages as the transparent distal region 111 of the probe 100 described previously.

The probe attachment may be made from any sufficiently robust and heat resistant material with insulating properties. Such materials include ceramics, although various plastics and composite materials may be found suitable. Preferably, the distal opening 310b has or is coated with a low coefficient of friction material such as Teflon® to suppress eschar build up and to prevent tissue, blood and mucous from adhering to the distal opening and impeding gas flow and effective treatment using the probe with the attachment.

In a manner similar to probe 100, probe attachment 300 may also include a marking on the lumen to indicate the orientation and position of the distal opening when in use. The marking may be of the kind illustrated in Figs. 4 and 5, or take another form which is meaningful to the surgeon.

Proximal end 301 a of the probe attachment includes fastening means (not shown) to fasten probe attachment 300 to the existing electrosurgical probe 320. The fastening means may take any suitable form as is known in the art. One example utilises a spring-loaded sleeve and pin type connector, commonly called a bayonet mount. Another method may utilise a close tolerance collar and stem assembly having a snap ring or locking collar to hold the probe attachment in place. Alternatively, a screw- type fitting may be used where one of the proximal end 301 a of the probe attachment and the end of the electrosurgical probe 320 have an inside or outside thread configured to engage the outside or inside thread of the other piece.

In one embodiment, the probe attachment includes pivot means akin to the pivot means 440 illustrated in Fig. 4. The pivot means may be incorporated into the fastening means, or may be provided in addition thereto. The pivot means may be in the form of a swivel or other rotatable coupling which permits the distal opening to rotate independently of the body of the probe lumen. The pivot means enables a physician to maintain contact between the distal opening of the probe attachment and the target tissue during movement of either the probe or target tissue.

In a manner similar to the probe 100, probe attachment is preferably adapted for use with an electrosurgical probe which is suitable for use when inserted in the working channel a flexible endoscope. Such endoscope may of the type used to treat the gastrointestinal tract or respiratory system.

The present invention in its various embodiments enables controlled contact-mode ablation of the target tissue, even when used endoscopically. Control is enhanced by the surgeon's ability to view the working tip of the device when distal region is transparent, or a transparent window is provided in the distal region of the lumen. This visualisation also enables the surgeon to recognise when the working tip is affected by blood, mucous or debris collected during a procedure and can prompt immediate remedial action by the surgeon cleaning the tip of the device. Cleaning is facilitated by the gas outlet. This enables continued effective ionisation of the inert gas within the lumen, and effective ablation of the target tissue with reduced carbonisation and smoke plume.

Thus the present invention provides a novel probe and probe attachment for use in electrosurgery. The design enables controlled directional contact between the distal opening of the probe/probe attachment and the target tissue during ablation which renders it safer for use in endoscopic procedures. When using the present invention for endoscopic surgery, a surgeon does not need to apply the same degree of caution as is necessary when using the prior art probes since the risk of embolism is reduced significantly.

When used in contact with the target tissue, the present invention also limits indiscriminate spread of high frequency current to the surrounding tissues. Confining the high frequency energy to the tissue exposed by the distal opening results in less energy loss to the surrounding tissues so the probe can be used at significantly lower power settings. For example, using the present invention, gentle ablation may be achieved using a power setting as low as 10 Watts compared with 40 Watts as is required to ablate tissue using the prior art probes.

The present embodiments do not require the same level of precaution as the prior art probes. The ease of use, particularly when the distal opening is maintained in contact with the target tissue eliminates much of the anxiety associated with use of traditional electrosurgical probes in the upper and lower gastrointestinal tracts, and in the bronchioles of lung transplant patients.

Although the present invention has been described with reference to the preferred embodiments illustrated in the accompanying drawings, it is to be understood that various modifications, additions and/or alterations may be made to the parts previously described without departing from the ambit of the present invention as defined in the claims appended hereto.

The word proximal as used herein refers to parts in closer proximity to the surgeon operating the probe and probe attachment. Following on from this, the word distal refers to parts located at a distance, relative to the surgeon.

The word surgeon as used herein is not intended to exclude other practitioners and users of the inventive probe and probe attachment. Thus, "surgeon" is intended to cover practitioners including but not limited to gastroenterologists, respiratory physicians and other personnel who are likely to use electrosurgery and endoscopic devices.

## Claims

1. A probe for use in electrosurgery, the probe including:
(a) a lumen (101; 301) having a proximal end (101a; 301 a) and a distal opening (101b; 301b; 410b);
(b) an electrode (103) within the lumen (101; 301), the electrode (103) having a proximal end (103a) connectable to a high frequency current source (105) and a discharge end (103b) recessed within the lumen (101; 301); and
**characterised by**:
(c) a gas outlet (107; 307; 407) in a distal region (111) of the lumen (101; 301) for releasing gas pressure (117) within the lumen (101; 301) when, in use, the distal opening (101b; 301b; 410b) of the lumen (101; 301) contacts target tissue (113), obstructing the gas flow in the lumen (101; 301) while the electrode (103) is activated, whereby to avoid gas embolism.

2. A probe according to claim 1, **characterised in that** the distal opening (101b; 301b; 410b) is shaped to create a desired treatment effect in the target tissue (113).

3. A probe according to claim 1 or 2, **characterised in that** the distal opening (101a; 301b; 410b) is oblique to facilitate treatment of a larger area of the target tissue (113).

4. A probe according to claim 3, **characterised in that** the gas outlet (107; 307; 407) is positioned such that the likelihood of the gas outlet (107; 307; 407) and the distal opening (101b; 301b; 410b) simultaneously contacting the target tissue (113) is minimised.

5. A probe according to any one of the preceding claims, **characterised in that** the gas outlet (107; 307; 407) is provided adjacent the discharge end (103b) of the electrode (103).

6. A probe according to any one of claims 1 to 4, **characterised in that** the gas outlet (107; 307; 407) is provided just distal of the discharge end (103b) of the electrode (103).

7. A probe according to any one of the preceding claims, **characterised in that** there is more than one gas outlet (107; 307; 407) for releasing gas pressure (117) within the lumen (101; 301).

8. A probe according to any one of the preceding claims, **characterised in that** the distal region (111) of the lumen (101; 301) includes a transparent portion.

9. A probe according to claim 8, **characterised in that** the transparent portion is manufactured from an insulating material.

10. A probe according to claim 8 or 9, **characterised in that** the transparent portion is ceramic.

11. A probe according to any one of the preceding claims, **characterised in that** the distal opening (101b; 301b; 410b) has a low coefficient of friction coating for suppressing eschar build up.

12. A probe according to any one of the preceding claims, **characterised by** a marking on the distal region (111) of the lumen (101; 301) to indicate orientation of the distal opening (101b; 301b; 410b) when in use.

13. A probe according to any one of the preceding claims, **characterised by** pivot means (440) for enabling the distal opening (101b; 301b; 410b) to maintain contact with the target tissue (113) during movement of the probe or target tissue (113).

14. A probe according to any one of the preceding claims, **characterised in that** gas within the lumen (101; 301) includes an ionisable gas.

15. A probe according to claim 14, **characterised in that** the ionisable gas is selected from the group including argon and helium.

16. A probe according to any one of the preceding claims, **characterised in that** a distal portion of the electrode (103) is tungsten with a highly conductive coating at its working tip.

17. A probe according to claim 16, **characterised in that** the highly conductive coating is silver.

18. A probe according to any one of the preceding claims, **characterised in that** it is adapted for use within a working channel of a flexible endoscope.

19. An endoscopic electrosurgery probe including a flexible lumen (101; 301) having a proximal end (101a; 301a) connectable to a source of ionisable gas (109) and a distal opening (101b; 301b; 410b), an electrode (103) having a proximal end (103a) attachable to a high frequency current source (105) and a discharge tip (103b) recessed within a distal region (111) of the lumen (101; 301), wherein the probe is activated by shrouding the electrode (103) in ionisable gas and applying high frequency current to the electrode (103); and **characterised by** the lumen (101; 301) also having a gas outlet (107; 307; 407) near the discharge tip (103b) of the electrode (103), wherein, in use, gas pressure (117) within the lumen (101; 301) is released through the gas outlet (107; 307; 407) when the distal opening (101b; 301b; 410b) contacts target tissue (113) obstructing gas flow in the lumen (101; 301) during activation of the electrode (103).

## Patentansprüche

1. Eine Sonde zum Gebrauch in der Elektrochirurgie, wobei die Sonde einschließt:
(a) ein Lumen (101; 301) mit einem proximalen Ende (101a; 301 a) und einer distalen Öffnung (101b; 301b; 410b);
(b) eine Elektrode (103) im Lumen (101; 301), wobei die Elektrode (103) ein proximales Ende (103a) hat, das koppelbar ist an eine Hochfrequenzstromquelle (105), und ein Entladungsende (103b), eingelassen im Lumen (101; 301); und **gekennzeichnet ist durch**:
(c) einen Gasauslass (107; 307; 407) in einer distalen Region (111) des Lumens (101; 301) zum Ablassen des Gasdrucks (117) in dem Lumen (101; 301), wenn, im Gebrauch, die distale Öffnung (101b; 301b; 410b) des Lumens (101; 301) Zielgewebe (113) kontaktiert, den Gasfluss im Lumen (101; 301) hemmend während die Elektrode (103) aktiviert ist, um **dadurch** Gasembolie zu vermeiden.

2. Eine Sonde nach Anspruch 1, **gekennzeichnet dadurch, dass** die distale Öffnung (101b; 301b; 410b) geformt ist, um einen gewünschten Behandlungseffekt im Zielgewebe (113) zu erreichen.

3. Eine Sonde nach Anspruch 1 oder 2, **gekennzeichnet dadurch, dass** die distale Öffnung (101b; 301b; 410b) schräg ist, um die Behandlung eines größeren Areals des Zielgewebes (113) zu erleichtern.

4. Eine Sonde nach Anspruch 3, **gekennzeichnet dadurch, dass** der Gasauslass (107; 307; 407) derart positioniert ist, dass die Wahrscheinlichkeit, dass der Gasauslass (107; 307; 407) und die distale Öffnung (101b; 301b; 410b) das Zielgewebe gleichzeitig kontaktieren, minimiert ist.

5. Eine Sonde nach einem der vorangehenden Ansprüche, **gekennzeichnet dadurch, dass** der Gasauslass (107; 307; 407) benachbart zum Entladungsende (103b) der Elektrode (103) vorgesehen ist.

6. Eine Sonde nach einem der Ansprüche 1 bis 4, **gekennzeichnet dadurch, dass** der Gasauslass (107; 307; 407) gerade distal des Entladungsendes (103b) der Elektrode (103) vorgesehen ist.

7. Eine Sonde nach einem der vorangehenden Ansprüche; **gekennzeichnet dadurch, dass** es mehr als einen Gasauslass (107; 307; 407) gibt, um den Gasdruck (117) in dem Lumen (101; 301) abzulassen.

8. Eine Sonde nach einem der vorangehenden Ansprüche, **gekennzeichnet dadurch, dass** die distale Region (111) des Lumens (101; 301) einen transparenten Teil beinhaltet.

9. Eine Sonde nach Anspruch 8, **gekennzeichnet dadurch, dass** der transparente Teil aus einem isolierenden Material hergestellt ist.

10. Eine Sonde nach Anspruch 8 oder 9, **gekennzeichnet dadurch, dass** der transparente Teil keramisch ist.

11. Eine Sonde nach einem der vorangehenden Ansprüche, **gekennzeichnet dadurch, dass** die distale Öffnung (101b; 301b; 410b) eine Beschichtung mit niedrigem Reibungskoeffizient zur Unterdrückung von Schorfbildung hat.

12. Eine Sonde nach einem der vorangehenden Ansprüche, **gekennzeichnet durch** eine Markierung auf der distalen Region (111) des Lumens (101; 301), um, im Gebrauch, die Ausrichtung der distalen Öffnung (101b; 301b; 410b) anzuzeigen.

13. Eine Sonde nach einem der vorangehenden Ansprüche, **gekennzeichnet durch** eine Zapfenverbindung (440), um der distalen Öffnung (101b; 301b; 410b) zu ermöglichen, Kontakt zu halten mit dem Zielgewebe (113) während der Bewegung der Sonde oder des Zielgewebes (113).

14. Eine Sonde nach einem der vorangehenden Ansprüche, **gekennzeichnet dadurch, dass** Gas in dem Lumen (101; 301) ionisierbares Gas beinhaltet.

15. Eine Sonde nach Anspruch 14, **gekennzeichnet dadurch, dass** das ionisierbare Gas ausgewählt ist aus der Gruppe, die Argon und Helium beinhaltet.

16. Eine Sonde nach einem der vorangehenden Ansprüche, **gekennzeichnet dadurch, dass** ein distaler Teil der Elektrode (103) Wolfram ist mit einer hoch leitfähigen Beschichtung an seiner Arbeitsspitze.

17. Eine Sonde nach Anspruch 16, **gekennzeichnet dadurch, dass** die hoch leitfähige Beschichtung Silber ist.

18. Eine Sonde nach einem der vorangehenden Ansprüche, **gekennzeichnet dadurch, dass** sie geeignet ist für den Gebrauch in einem Arbeitskanal eines flexiblen Endoskops.

19. Eine endoskopische elektrochirurgische Sonde, ein flexibles Lumen (101; 301) beinhaltend, das ein proximales Ende (101 a; 301 a) hat, das koppelbar ist an eine Quelle ionisierbaren Gases (109) und eine distale Öffnung (101b; 301b; 410b) hat, eine Elektrode (103) mit einem proximalen Ende (103a), ansteckbar an eine Hochfrequenzstromquelle (105) und eine Entladungsspitze (103b), eingelassen in eine distale Region (111) des Lumens (101; 301), wobei die Sonde aktiviert wird durch Eintauchen der Elektrode (103) in ionisierbares Gas und Anwenden hochfrequenten Stroms auf die Elektrode (103); und **gekennzeichnet durch** das Lumen (101; 301) mit ebenfalls einem Gasauslass (107; 307; 407) nahe der Entladungsspitze (103b) der Elektrode (103), wobei, im Gebrauch, Gasdruck (117) im Lumen (101; 301) **durch** den Gasauslass (107; 307; 407) abgelassen wird, wenn die distale Öffnung (101b; 301b; 410b) Zielgewebe (113) kontaktiert, den Gasfluss im Lumen (101; 301) während der Aktivierung der Elektrode (103) hemmend.

## Revendications

1. Sonde destinée à être utilisée en électrochirurgie, la sonde comprenant :
(a) une lumière (101 ; 301) ayant une extrémité proximale (101a ; 301a) et une ouverture distale (101b ; 301b ; 410b) ;
(b) une électrode (103) à l'intérieur de la lumière (101 ; 301), l'électrode (103) ayant une extrémité proximale (103a) connectable à une source de courant haute fréquence (105) et une extrémité de décharge (103b) logée à l'intérieur de la lumière (101 ; 301) ; et
**caractérisée par** :
(c) une sortie des gaz (107 ; 307 ; 407) dans une région distale (111) de la lumière (101 ; 301) pour évacuer la pression gazeuse (117) à l'intérieur de la lumière (101 ; 301) lorsque, lors de l'utilisation, l'ouverture distale (101b ; 301b ; 410b) de la lumière (101 ; 301) entre en contact avec le tissu cible (113), bloquant l'écoulement gazeux dans la lumière (101 ; 301) alors que l'électrode (103) est activée, permettant ainsi d'éviter une embolie gazeuse.

2. Sonde selon la revendication 1, **caractérisée en ce que** l'ouverture distale (101b ; 301b ; 410b) est façonnée pour créer un effet de traitement souhaité dans le tissu cible (113).

3. Sonde selon la revendication 1 ou 2, **caractérisée en ce que** l'ouverture distale (101b ; 301b ; 410b) est oblique pour faciliter le traitement d'une plus grande zone du tissu cible (113).

4. Sonde selon la revendication 3, **caractérisée en ce que** la sortie des gaz (107 ; 307 ; 407) est positionnée de telle sorte que la probabilité pour que la sortie des gaz (107 ; 307 ; 407) et l'ouverture distale (101b ; 301b ; 410b) soient simultanément en contact avec le tissu cible (113) est minimisée.

5. Sonde selon l'une quelconque des revendications précédentes, **caractérisée en ce que** la sortie des gaz (107 ; 307 ; 407) est disposée de façon adjacente à l'extrémité de décharge (103b) de l'électrode (103).

6. Sonde selon l'une quelconque des revendications 1 à 4, **caractérisée en ce que** la sortie des gaz (107 ; 307 ; 407) est disposée simplement de façon distale à l'extrémité de décharge (103b) de l'électrode (103).

7. Sonde selon l'une quelconque des revendications précédentes, **caractérisée en ce qu'**il existe plus d'une sortie des gaz (107 ; 307 ; 407) pour évacuer la pression gazeuse (117) à l'intérieur de la lumière (101 ; 301).

8. Sonde selon l'une quelconque des revendications précédentes, **caractérisée en ce que** la région distale (111) de la lumière (101 ; 301) comprend une partie transparente.

9. Sonde selon la revendication 8, **caractérisée en ce que** la partie transparente est fabriquée à partir d'un matériau isolant.

10. Sonde selon la revendication 8 ou 9, **caractérisée en ce que** la partie transparente est de la céramique.

11. Sonde selon l'une quelconque des revendications précédentes, **caractérisée en ce que** l'ouverture distale (101b ; 301b ; 410b) a un revêtement à faible coefficient de frottement pour supprimer la formation d'escarres.

12. Sonde selon l'une quelconque des revendications précédentes, **caractérisée par** un marquage sur la région distale (111) de la lumière (101 ; 301) pour indiquer l'orientation de l'ouverture distale (101b ; 301b ; 410b) lorsqu'elle est utilisée.

13. Sonde selon l'une quelconque des revendications précédentes, **caractérisée par** des moyens de pivotement (440) pour permettre à l'ouverture distale (101b ; 301b ; 410b) de maintenir le contact avec le tissu cible (113) lors du mouvement de la sonde ou du tissu cible (113).

14. Sonde selon l'une quelconque des revendications précédentes, **caractérisée en ce que** le gaz à l'intérieur de la lumière (101 ; 301) comprend un gaz ionisable.

15. Sonde selon la revendication 14, **caractérisée en ce que** le gaz ionisable est choisi dans le groupe comprenant l'argon et l'hélium.

16. Sonde selon l'une quelconque des revendications précédentes, **caractérisée en ce qu'**une partie distale de l'électrode (103) est en tungstène avec un revêtement fortement conducteur au niveau de sa pointe de travail.

17. Sonde selon la revendication 16, **caractérisée en ce que** le revêtement fortement conducteur est de l'argent.

18. Sonde selon l'une quelconque des revendications précédentes, **caractérisée en ce qu'**elle est conçue pour être utilisée à l'intérieur d'un canal de travail d'un endoscope flexible.

19. Sonde électrochirurgicale endoscopique comprenant une lumière flexible (101 ; 301) ayant une extrémité proximale (101a ; 301a) connectable à une source de gaz ionisable (109) et une ouverture distale (101b ; 301b ; 410b), une électrode (103) ayant une extrémité proximale (103a) attachable à une source de courant haute fréquence (105) et une pointe de décharge (103b) logée à l'intérieur d'une région distale (111) de la lumière (101 ; 301), la sonde étant activée par enveloppement de l'électrode (103) dans du gaz ionisable et par application d'un courant haute fréquence à l'électrode (103) ; et **caractérisée en ce que** la lumière (101 ; 301) a également une sortie des gaz (107 ; 307 ; 407) près de la pointe de décharge (103b) de l'électrode (103), dans laquelle, en cours d'utilisation, la pression des gaz (117) à l'intérieur de la lumière (101 ; 301) est évacuée à travers la sortie des gaz (107 ; 307 ; 407) lorsque l'ouverture distale (101b ; 301b ; 410b) entre en contact avec le tissu cible (113) bloquant l'écoulement gazeux dans la lumière (101 ; 301) lors de l'activation de l'électrode (103).
